(19) Europäisches Patentamt
European Patent Office
Office européen des brevets

(11) **EP 2 291 650 B1**

(12) **EUROPÄISCHE PATENTSCHRIFT**

(45) Veröffentlichungstag und Bekanntmachung des Hinweises auf die Patenterteilung:
**18.09.2013   Patentblatt 2013/38**

(51) Int Cl.:
***G01N 33/22*** *(2006.01)*

(21) Anmeldenummer: **09765455.2**

(86) Internationale Anmeldenummer:
**PCT/DE2009/000855**

(22) Anmeldetag: **18.06.2009**

(87) Internationale Veröffentlichungsnummer:
**WO 2009/152819 (23.12.2009 Gazette 2009/52)**

(54) **VERFAHREN ZUR BESTIMMUNG DER GASQUALITÄT EINES ZUMINDEST TEILWEISE MIT BIOGAS ODER AUFBEREITETEM BIOGAS VERSETZTEN PROBENGASES**

METHOD FOR DETERMINING THE GAS QUALITY OF A SAMPLE GAS AT LEAST PARTIALLY MIXED WITH BIOGAS OR PROCESSED BIOGAS

PROCÉDÉ DE DÉTERMINATION DE LA QUALITÉ D'UN GAZ D'ÉCHANTILLONNAGE AU MOINS PARTIELLEMENT ADDITIONNÉ DE BIOGAZ OU DE BIOGAZ PURIFIÉ

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **21.06.2008   DE 102008029553**

(43) Veröffentlichungstag der Anmeldung:
**09.03.2011   Patentblatt 2011/10**

(73) Patentinhaber: **Elster GmbH**
**44357 Dortmund (DE)**

(72) Erfinder: **KASTNER, Joachim**
**44225 Dortmund (DE)**

(74) Vertreter: **Schneider, Uwe**
**Patentanwalt**
**Holbeinstrasse 27**
**59423 Unna (DE)**

(56) Entgegenhaltungen:
WO-A1-03/062618          DE-A1- 10 121 641
US-A1- 2004 195 531

**Beschreibung**

Die Erfindung betrifft ein Verfahren zur Bestimmung der Gasqualität eines zumindest teilweise mit Biogas oder aufbereitetem Biogas versetzten Probengases gemäß Oberbegriff des Anspruches 1.

Biogas aus Biomasse stellt einen regenerativen, $CO_2$-neutralen Energieträger dar. Die Biogaswirtschaft wird daher in Deutschland und der EU öffentlich gefördert.

Eine weitverbreitete und sehr effiziente Verwendung von Biogas ist die Verbrennung in Blockheizkraftwerken. Durch die Kraft-Wärme-Kopplung wird ein hoher Gesamtwirkungsgrad erreicht, sofern es eine sinnvolle Nutzung für die Abwärme gibt. Falls die Wärme am Ort der Biogaserzeugung nicht gebraucht wird, ist es vom Gesamtwirkungsgrad effizienter, das Biogas zum Ort der Wärmenutzung zu transportierten und dort zu verbrennen. Der Transport kann durch Einspeisung in das Erdgasnetz erfolgen. Die Bedingungen für die Gasqualität und die Abrechnungsmessung wurden in Zusammenarbeit der Erd- und Biogaswirtschaft in der G262 [2] geregelt.

Biogas wird durch fermentative oder thermische Prozesse erzeugt. Bei fermentativer Erzeugung wird organisches Material (Müll, Gülle, Energiepflanzen (NaWaRo = Nachwachsende Rohstoffe), Klärschlamm, tierisches Material) durch Bakterien zu Gas zersetzt. Die entstehenden Gase enthalten als Leitkomponenten $CH_4$ und $CO_2$ (siehe Tabelle 1):

**Tabelle 1 - Beschaffenheit der Gase aus Bio-, Klär- und Deponiegasanlagen (Anhaltswerte)**

| Herkunft | Hauptkomponenten | | | Gasbegleitstoffe**) | | | $H_2O$- | Betriebsdaten der Anlage | |
|---|---|---|---|---|---|---|---|---|---|
| | $CH_4$ Vol-% | $CO_2$ Vol-% | $O_2/N_2$ Vol-% | KW*) $mg/m^3$ | $H_2S$ $mg/m^3$ | FCKW*) $mg/m^3$ | Taupunkt °C | $P_n$ mbar | t °C |
| Biogasanlagen | 50-85 | 50-15 | Rest | - | bis ca. 10 000 | - | ca. 35 | bis ca. 50 | ca. 35 |
| Klärgasanlagen | 65-70 | 35-20 | Rest | bis ca. 10 | bis ca. 10 000 | - | ca. 35 | bis ca. 50 | ca. 35 |
| Mülldeponie | 40-60 | 40-20 | Rest | bis ca. 300 | bis ca. 900 | 20 bis ca. 1000 | ca. 25 | 0 bis 3 | ca. 25 |

*) KW = Kohlenwasserstoffe (C-Zahl > 2); FCKW = Fluor-Chlor-Kohlenwasserstoffe
**) Abhängig von einer evtl. Kofermentation können zusätzlich andere Gasbegleitsoffe auftreten

Bei der thermischen Erzeugung wird das Biomaterial durch Erhitzung mit Luft als Vergasungsmittel oder unter Luftabschluss (Pyrolyse) vergast. Die Leitkomponenten dieser Synthesegase sind $H_2$, CO, $CO_2$, $N_2$ (siehe Tabelle 2, G262 [2])

**Tabelle 2 - Beschaffenheit (Hauptkomponenten) von Gasen aus thermischen Gaserzeugungsverfahren (Anhaltswerte, überarbeiteter Auszug aus [Lit. 10])**

| Vergasungsverfahren und Vergasungsmittel | Brennwert $MJ/m^3$ | Zusammensetzung | | | | | | |
|---|---|---|---|---|---|---|---|---|
| | | $H_2$ Vol-% | CO Vol-% | $CH_4$ -Vol-% | $CO_2$ Vol-% | $N_2$ Vol-% | $H_2O$ Vol-% | KW Vol-% |
| Festbett: Sauerstoff (FbS) | 9,3-9,7 | 35-43 | 13 - 34 | 0-12 | 19-35 | 0-2 | bis 3 | 0-3 |
| Wirbelschicht: Luft (WbL) | 5-7 | 4-20 | 9-22 | 1-7 | 14-16 | bis 56 | 0-3 | 0-2 |
| Wirbelschicht: Sauerstoff (WbS) | 9-12 | 40-45 | 29-34 | bis 3 | 19-22 | 0-2 | bis 3 | bis 2 |
| Flugstromverfahren: Sauerstoff (FeS) | 10-12 | 30-35 | 50-70 | bis 1 | 2-15 | 0-2 | bis 3 | bis 1 |
| Pyrolyse (Pyr) | 7-15 | 4-46 | 18-40 | bis 15 | 15-20 | 0 | < 1 | bis 4 |

Die thermische Erzeugung hat bisher nur begrenzte Anwendung gefunden. In einer aktuellen Studie [1] wird jedoch die thermo-chemische Erzeugung von Biogas als neue Option genannt, um das Biogaspotential durch eine bessere Verfügbarkeit des Rohstoffs Holz erheblich zu vergrößern. Das entstehende Synthesegas soll in einem nachgeschalteten katalytischen Methanisierungsprozess auf Einspeisequalität gebracht werden. Angaben zur Gasbeschaf-fenheit, insbesondere zur $H_2$-Konzentration werden in der Studie nicht gemacht.

Die Aufbereitung und Analytik der Rohsynthesegase ist auf Grund der zahlreichen Leitkomponenten komplexer als bei den fermentativen Gasen. Sowohl für die Verwendung vor Ort als auch für die Einspeisung in Gastransportnetze muss das Rohbiogas aufbereitet werden, damit keine Störung oder Schädigung der benutzten Anlagen erfolgt und bei der Verbrennung keine gesundheitlich oder ökologisch schädliche Produkte entstehen. Die Anforderungen an die Gasqualität für die Einspeisung sind in der G262 [2] geregelt. Demnach darf Biogas bei entsprechender Aufbereitung als Zusatz- oder Aüstauschgas in die öffentliche Gasversorgung eingespeist werden.

Bei Einspeisung in H-Gasnetze (außer russisches Gas) muss das aufbereitete Biogas ($H<11,064$ kWh/m$^3$) durch Zumischung von Flüssiggas (LPG=Liquid Petroleum Gas, Propan/Butan) konditioniert werden. In diesem Fall erfolgt die eichfähige Messung nach der Flüssiggaszumischung. Die Messung dient hierbei vornehmlich der Gewährleistung der jeweiligen Gasbeschaffenheit im Gasnetz, die sich durch die Zumischung des Biogases ändern kann sowie zur Abrechnung der eingespeisten Gasmengen im Hinblick auf die damit erzeugbaren Wärmemengen.

Die bisherigen Bauartzulassungen für handelsübliche Gasbeschaffenheitsmessgeräte decken nicht automatisch die Messung von Biogas ab. Problematisch ist hierbei vor allem der Effekt von Wasserstoff $H_2$, der nicht vollständig ausgeschlossen werden kann (typischerweise 125...250ppm, viel 500...700ppm, Spitze 2000ppm).

Nach G262 [2] muss das Biogas für die Einleitung in öffentliche Gastransportnetze eichrechtlich gemessen werden. Dies betrifft sowohl die Volumenmessung als auch die Messung der Gasbeschaffenheit.

Zunächst wird unterschieden zwischen der Messtechnik für die eichamtliche Energiemessung und für die Überwachung von Parametern der Produktqualität ($xO_2$, $xH_2S$). Im Idealfall werden bei Messaufgaben in einem Gerät integriert.

Zur Messung der Gasbeschaffenheit sind im Laufe der Jahre viele Geräte und Messverfahren entwickelt worden, wobei zunehmend Geräte und Verfahren zum Einsatz kommen, bei denen die Absorption des Gases in Bezug auf Infrarotstrahlung genutzt wird. Derartige Geräte und Verfahren sind beispielsweise aus der EP 1 141 677 B1 und der 1 147 396 B1 bekannt. Hierbei kommen korrelative Verfahren zum Einsatz, mit deren Hilfe die gemessene Absorption der Infrarotstrahlung auf die jeweiligen Größen des Gases umgerechnet und damit betriebsspezifische Größen des Gases bestimmt werden können. Zu den Einzelheiten derartiger Verfahren wird ausdrücklich auf die EP 1 141 677 B1 und die 1 147 396 B1 verwiesen.

Problematisch an der Nutzung derartiger Geräte und Verfahren für die Messung der Gasbeschaffenheit von Biogas ist es, dass das Biogas kleine Mengen an $H_2$ enthalten kann. Zwar sollten Fermentergase frei von $H_2$ sein (siehe Tabelle 1, G262 [2]), aus der Praxis ist aber bekannt, dass aufbereitete Biogase 150...500 ppm, in Spitzen 2000 ppm $H_2$ enthalten können. Dementsprechend fordert die Physikalisch-Technische Bundesanstalt (PTB) für die Zulassung von Biogas für die Einspeisung in Gasnetze eine Untersuchung mit entsprechender $H_2$-Beimischung.

Die bisher verwendeten korrelativen Verfahren basieren auf einer Wärmeleitfähigkeitsmessung, die empfindlich auf die hohe Wärmeleitfähigkeit von $H_2$ reagiert.

Aus der WO 03/062618 A1 zeigt ein Verfahren zur Bestimmung der Qualität eines Brenngases, bei dem die Anteile von $CH_4$, LPG und $CO_2$ durch Messung der jeweiligen optischen Absorption bestimmt werden und der Anteil von $N_2$ durch Messung der Wärmeleitfähigkeit. Aus der US 2004/195531 A1 ist bekannt, den $H_2$-Anteil in einem Glei-chungssystem zu berücksichtigen, in dem alle zu bestimmenden Stoffmengenanteile die Wärmeleitfähigkeit beeinflussen und zusammen 100 % ergeben.

Aufgabe der vorliegenden Erfindung ist es daher, die gattungsgemäßen Verfahren für die Messung der Gasbeschaffenheit derart weiter zu entwickeln, dass sie auch zur Messung von Biogas oder aufbereitetem Biogas geeignet sind, ohne dass Wasserstoff $H_2$ oder andere in Biogas vorhandene oder zum Biogas hinzugefügte Beimengungen wie etwa Sauerstoff $O_2$ oder Flüssiggas LPG zu unzulässigen Veränderungen der Messwerte führen können.

Die Lösung der erfindungsgemäßen Aufgabe ergibt sich aus den kennzeichnenden Merkmalen des Anspruches 1 in Zusammenwirken mit den Merkmalen des Oberbegriffes. Weitere vorteilhafte Ausgestaltungen der Erfindung ergeben sich aus den Unteransprüchen.

Die Erfindung geht aus von einem Verfahren zur Bestimmung der Gasqualität eines zumindest teilweise mit Biogas oder aufbereitetem Biogas versetzten Probengases aufweisend die Hauptkomponenten $CH_4$, $CO_2$, $N_2$, $O_2$, $H_2$, ausgehend von einem unter Betriebsbedingungen mittels infrarotspektroskopischer Messverfahren bestimmten Spektrum des Probengases, aus dem mittels korrelativer Verfahren die Stoffmengenanteile des Probengases bestimmt und in Kenngrößen der Gasqualität umgerechnet werden. Ein derartiges gattungsgemäßes Verfahren wird dadurch weiter gebildet, dass die optische Absorption von Methan $CH_4$ und von Kohlendioxid $CO_2$ und die Wärmeleitfähigkeit $\lambda$ des Probengases und bei Zumischung von Flüssiggas LPG die optische Absorption des LPG gemessen wird, aus der Absorption des $CH_4$ der Stoffmengenanteil $xCH_4$ und aus der Absorption des $CO_2$ der Stoffmengenanteil $xCO_2$ bestimmt wird, aus den Stoffmengenanteilen $xCH_4$, $xCO_2$ und der Wärmeleitfähigkeit $\lambda$ und bei Zumischung von Flüssiggas LPG dem Stoffmengenanteil $xLPG$ mittels einer Korrelationsrechnung $\lambda = F\,(xCH_4,\ xCO_2,\ xN_2,\ xO_2,\ xH_2)$, bzw. $\lambda = F\,(xCH_4,$

$xCO_2$, $xN_2$, $xO_2$, $xH_2$, $xLPG$) die optisch nicht erfassten Stoffmengenanteile des Stickstoffs $xN_2$, des Sauerstoffs $xO_2$ und des Wasserstoffs $xH_2$ bestimmt werden, woraufhin aus den so gewonnenen Stoffmengenanteilen charakteristische Parameter des Probengases berechnet werden. Besonders vorteilhaft ist bei dieser Vorgehensweise, dass auf Basis der messbaren Werte für die Bestandteile Methan $CH_4$ und von Kohlendioxid $CO_2$ des Probengases und der Messung seiner Wärmeleitfähigkeit $\lambda$ und bei Zumischung von Flüssiggas LPG der optischen Absorption des LPG anhand der Korrelationsrechnung durch einen einfachen linearen Ansatz die Stoffmengenanteile von Stickstoff $N_2$, Sauerstoff $O_2$ und Wasserstoff $H_2$ dadurch ermittelt werden können, indem bei einem linearen Ansatz der Anteil des Wasserstoffs $H_2$ direkt analytisch berechnet werden kann (oder auch bei einem nichtlinearen Ansatz die Korrelationsrechnung solange ausgeführt wird, bis der aus der Korrelationsrechnung hervorgehende Wert für die Wärmeleitfähigkeit $\lambda$ dem gemessenen Wert entspricht) . Anhand des linearen Ansatzes lassen sich somit die bis dahin unbekannten Stoffmengenanteile von Stickstoff $N_2$, Sauerstoff $O_2$ und Wasserstoff $H_2$ am Probengas und daraus charakteristische Größen des Probengases wie etwa Brennwert, Heizwert, Dichte, Wobbe- Index, Methanzahl oder dgl. analytisch bestimmen. Der lineare Ansatz für die Korrelation der Gasbestandteile ist dabei einfach und damit schnell durchführbar und benötigt nur überschaubare Rechenleistung. Als Messwerte werden nur die Werte für die Absorption von Methan $CH_4$ und von Kohlendioxid $CO_2$ und die Wärmeleitfähigkeit $\lambda$ des Probengases und bei Zumischung von Flüssiggas LPG noch die optische Absorption des LPG benötigt. Bei alternativer Verwendung eines nichtlinearen Ansatzes und dabei notwendiger numerischer Lösung werden Startwerte für die Stoffmengenanteile von Stickstoff $xN_2$ und Sauerstoff $xO_2$ benötigt, anhand derer ein Startwert für den Stoffmengenanteil des Wasserstoffs $H_2$ berechnet werden kann. Anhand dieser Startwerte und der gemessenen Werte kann dann iterativ durch Anpassung der Werte für Stoffmengenanteile von Stickstoff $xN_2$ und Sauerstoff $xO_2$ die Korrelationsrechnung ausgeführt und jeweils durch den Vergleich von berechneter Wärmeleitfähigkeit $\lambda$ und gemessener Wärmeleitfähigkeit $\lambda_m$ angepasst werden. Stimmen die Werte berechneter Wärmeleitfähigkeit $\lambda$ und gemessener Wärmeleitfähigkeit $\lambda_m$ überein, liegen die tatsächli- _ chen Stoffmengenanteile von Stickstoff $N_2$, Sauerstoff $O_2$ und Wasserstoff $H_2$ vor und daraus können dann die weiteren charakteristischen Größen des Probengases anhand der physikalischen Gesetze berechnet werden.

Von Vorteil für die Durchführung des Verfahrens ist es, wenn für die Korrelation $\lambda = F$ ($xCH_4$, $xCO_2$, $xN_2$, $xO_2$, $xH_2$) ein linearer Ansatz aus den Stoffmengenanteilen gewählt wird:

$$\lambda = \lambda_0 + xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2$$

Ein solcher Ansatz ist rechentechnisch einfach und analytisch durchführbar und benötigt eine relativ geringe Rechenleistung. Dadurch ist dieser Ansatz im Betrieb schnell ausführbar und die Ergebnisse der Korrelation und damit die zu bestimmenden charakteristischen Größen stehen schnell zur Verfügung.

Die Parameter $\lambda_0$, $\lambda CH_4$, $\lambda CO_2$, $\lambda(N_2, O_2)$, $\lambda H_2$, sind dabei die allgemein formulierten Fitparameter der linearen Modellierung und können wie folgt bestimmt werden: Für eine Reihe von Gasen mit bekannter Zusammensetzung und bekannter ReferenzWärmeleitfähigkeit wird die Modellwärmeleitfähigkeit nach dem linearen Ansatz berechnet. Die Fitparameter werden dabei vorteilhaft so optimiert, dass sich für alle Gase eine minimale Abweichung zwischen den Modell- und Referenzwärmeleitfähigkeiten ergibt. Die Parameter $\lambda CH_4$, $\lambda CO_2$, $\lambda(N_2,O_2)$, $\lambda H_2$ können als Wärmeleitfähigkeiten der jeweiligen Reinstoffe bzw. Stoffgruppe interpretiert werden. Der Parameter $\lambda_0$ ist dabei die eine Ursprungswärmeleitfähigkeit des linearen Modells und erlaubt, dass die Modellfunktion nicht durch den Koordinatenursprung laufen muss (Achsenabschnitt).

 Alternativ kann bei einer Zumischung von LPG zu dem Probengas für die Korrelation $\lambda = F$ ($xCH_4$, $xCO_2$, $xN_2$, $xO_2$, $xH_2$, $xLPG$) ebenfalls ein linearer Ansatz aus den Stoffmengenanteilen gewählt werden:

$$\lambda = xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2 + xLPG \cdot \lambda LPG$$

Auch ein solcher Ansatz lässt sich analytisch berechnen und gibt daher schnell und einfach Ergebnisse für die benötigten Größen des Probengases.

Von Vorteil für die Durchführung des Verfahrens kann es sein, wenn für die Korrelation $\lambda = F$ ($xCH_4$, $xCO_2$, $xN_2$, $xO_2$, $xH_2$) , bzw. $\lambda = F$ ($xCH_4$, $xCO_2$, $xN_2$, $xO_2$, $xH_2$, $xLPG$) ein Polynomansatz mit Termen höherer Ordnung und Mischtermen aus den Stoffmengenanteilen gewählt wird. Ein derartiger Polynomansatz ist zwar gegenüber dem vorstehenden Ansatz aufwändiger auszurechnen, doch ergibt sich möglicherweise eine höhere Genauigkeit der Ergebnisse. Hier kann die Lösung des Ansatzes der Korrelation über einen Polynomansatz durch numerische Iteration erfolgen.

Für beide Ansätze der Korrelationsrechnung ist es von Vorteil, wenn die gemessene Wärmeleitfähigkeit $\lambda_m$ und die berechnete Wärmeleitfähigkeit $\lambda$ durch iterative Variation und Berechnung der unbekannten Stoffmengenanteile $xN_2$, $xO_2$, $xH_2$ miteinander verglichen werden. Hierbei ist die im wesentlichen passende Übereinstimmung der gemessenen Wärmeleitfähigkeit $\lambda_m$ und der berechneten Wärmeleitfähigkeit $\lambda$ das Kriterium, anhang derer die Korrelationsrechnung beendet werden kann. Liegt eine Übereinstimmung der gemessenen Wärmeleitfähigkeit $\lambda_m$ und der berechneten Wärmeleitfähigkeit $\lambda$ vor, kann durch Rückrechnung anhand des Ansatzes der Korrelationsrechnung die genaue Stoffmengenverteilung der nicht messtechnisch erfassten Bestandteile des Probengases berechnet und daraus dann die charakteristischen Größen ermittelt werden.

Von Vorteil ist es weiterhin bei der Durchführung der Korrelationsrechnung, wenn die Stoffmengenanteile $xN_2$ von $N_2$ und $xO_2$ von $O_2$ als Summe x $(N_2, O_2)$ : = $xN_2 + xO_2$ aus der Korrelation bestimmt werden, da sich die Bestandteile Sauerstoff und Stickstoff ohnehin nur schwer getrennt voneinander erfassen lassen.

Das Verfahren kann auch dadurch weiter gebildet werden, dass die Wärmeleitfähigkeit des Probengases bei zwei Temperaturen ($\lambda1$, $\lambda2$) gemessen wird, die Wärmekapazität des Probengases bei zwei Temperaturen (C1, C2) gemessen wird und die Stoffmengenanteilen $xCH_4$, $xCO_2$, $xN_2+xO_2$, $xH_2$ durch Lösung eines Systems von Korrelationsgleichungen

$$\lambda1 = F1 (xCH_4, xCO_2, xN_2 + xO_2, xH_2)$$

$$\lambda2 = F2 (xCH_4, xCO_2, xN_2 + xO_2, xH_2)$$

$$C1= F3 (xCH_4, xCO_2, xN_2 + xO_2, xH_2)$$

$$C2 = F4 (xCH_4, xCO_2, xN_2 + xO_2.xH_2)$$

bestimmt werden. Durch die Verwendung weiterer Messgrößen sowie ggf. eines nichtlinearen Ansatzes für die Korrelation lassen sich weitere Einflussgrößen für die Korrelation einbeziehen, die die Temperaturabhängigkeit der Wärmekapazität und der Wärmeleitfähigkeit des Probengases ausnutzen und damit ggf. eine separate Bestimmung der Stoffmengenanteile von Stickstoff $xN_2$ und Sauerstoff $xO_2$ erlauben. Dieser Ansatz ist zwar messtechnisch und rechentechnisch für die Korrelation aufwändiger, gleichzeitig aber kann durch die größere Informationsmenge der gemessenen Werte die Anzahl der Iterationsschritte bei der Korrelation verringert werden. Auch hierbei können nach Feststellen einer Übereinstimmung von berechneter Wärmeleitfähigkeit $\lambda$ und gemessener Wärmeleitfähigkeit $\lambda_m$ die schon vorstehend beschriebenen charakteristischen Größen des Probengases berechnet werden.

Von Vorteil ist es weiterhin, wenn der spektrale Filter zur Bestimmung der Anteile der Kohlenwasserstoffe im Bereich von im wesentlichen 3,33 $\mu$m arbeitet und nicht bei den für Probengase aus reinen Kohlenwasserstoffen sonst üblichen 3,46 $\mu$m arbeitet, da dann die Absorption der Infrarotstrahlung in dem Biogas besonders günstig ist.

Weiterhin ist es von Vorteil für die Betriebssicherheit, wenn die Gasqualität in Bezug auf die Konzentration von Sauerstoff $O_2$ und Schwefelwasserstoff $H_2S$ überwacht wird. In Gasleitungssystemen dürfen die Werte für diese Verunreinigungen des Gases bestimmte Werte nicht überschreiten, um z.B. die Betriebssicherheit des Gastransportes bzw. die Lebensdauer des Gasleitungssystems nicht negativ zu beeinflussen. Daher können diese Werte z.B. mittels chemischer Sensoren, insbesondere mittels elektrochemischer Zellen auf das Überschreiten bestimmter Grenzwerte überwacht werden. Ebenfalls ist es denkbar, dass die Überwachung des Probengases auf die Konzentration von Sauerstoff $O_2$ mittels Laserdioden durchgeführt wird.

Eine besonders bevorzugte Ausführungsform der erfindungsgemäßen Verfahren für die Lösung mittels numerischer Iteration bei Verwendung eines nicht-linearen Ansatzes zeigt die Zeichnung.

Es zeigen:

Figur 1 -   Ablaufdiagramm des Verfahrens für die Lösung mittels numerischer Iteration bei Verwendung eines nichtlinearen Ansatzes,

Figur 2 -   Ablaufdiagramm des Verfahrens für die Lösung mittels numerischer Iteration bei Verwendung eines nichtlinearen Ansatzes bei Zumischung von Flüssiggas zu dem Probengas.

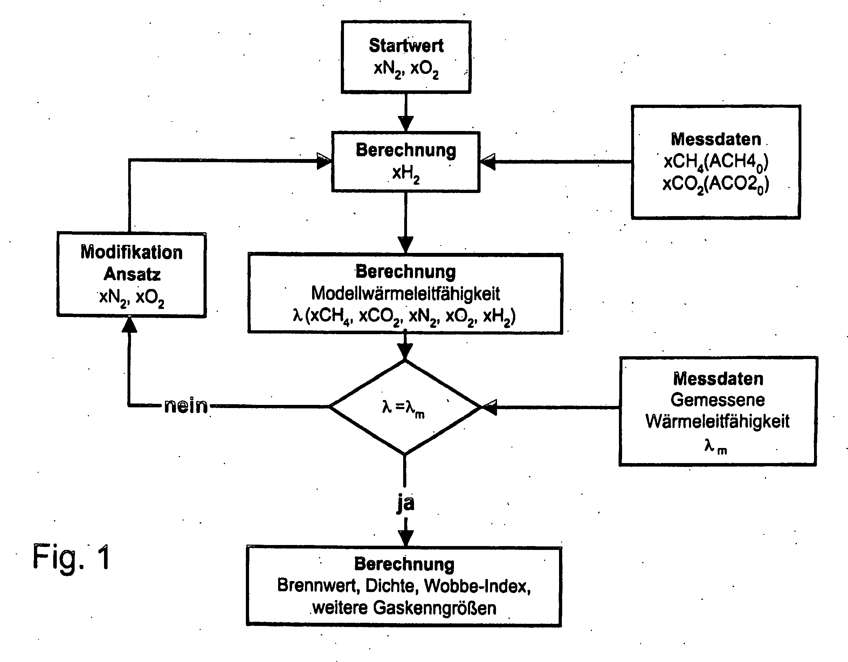

Die Figur 1 beschreibt den grundsätzlichen Ablauf des Verfahrens mit einer Kohlenwasserstoff-Komponente (nur $CH_4$) gemäß Anspruch 1 für die Lösung mittels numerischer Iteration bei Verwendung eines nicht-linearen Ansatzes.

Hierfür sind für die Korrelationsrechnung an sich vorab folgende physikalischen Grundlagen zu formulieren:

Für die Messung von Biogas in Einspeisequalität nach DVGW-G260, -G262 ohne LPG-Zumischung kann folgender Ansatz verwendet werden. Es gilt die Normierung:

$$xCH_4 + xCO_2 + xN_2 + xO_2 + xH_2 = 1 \qquad \textbf{Gl.1}$$

Brennwert und Dichte werden wie folgt berechnet:

$$H = xCH_4 \cdot HCH_4 + xH_2 \cdot HH2 \qquad \textbf{Gl. 2}$$

$$\rho = xCH_4 \cdot \rho CH_4 + xCO_2 \cdot \rho CO_2 + xN_2 \cdot \rho N_2 + xO_2 \cdot \rho O_2 + xH_2 \cdot \rho H_2 \qquad \textbf{Gl. 3}$$

Die Molenbrüche $xCH_4$ und $xCO_2$ werden nach dem Beer-Lambert-Gesetz direkt aus optischen Absorptionsmessungen bestimmt. Dabei müssen gegebenenfalls spezielle, vom reinen Beer-Lambert-Gesetz abweichende Kennlinien berücksichtigt werden (F1, F2 sind empirische Kalibrierfunktionen):

$$xCH_4 = F1 (ACH4_0) \qquad \textbf{Gl. 4}$$

$$xCO_2 = F2 (ACO2_0) \qquad \textbf{Gl. 5}$$

$ACH4_0$ und $ACO2_0$ sind die optischen Absorptionen bezogen auf einen Referenzzustand $(p_0, T_0)$. Geeignete Absorptionsbanden liegen im infraroten Spektralbereich; typische Bereiche sind: Kohlenwasserstoffe 3, 1- 3, 6 $\mu m$, $CO_2$ 4, 2- 4, 4 $\mu m$.

Die Konzentrationen von $H_2$, $N_2$ und $O_2$ können aus der Messung der Wärmeleitfähigkeit $\lambda$, der Normierungsbedingung Gl. 1 und folgender Modellrechnung bestimmt werden. Für die Wärmeleitfähigkeit des Gases wird ein linearer Mischungsansatz gemacht:

$$\lambda = xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2 \qquad \textbf{Gl. 6}$$

Die Konzentrationen $xN_2$ und $xO_2$ lassen sich nur als Summe bestimmen, die Terme werden daher zusammengefasst zu $x(N_2, O_2):=x(N_2) + x(O_2)$

Die Wärmeleitfähigkeit des Teilgemisches $(N_2, O_2)$ wird mit $\lambda(N_2, O_2)$ bezeichnet.

Gleichung 6 lautet damit:

$$\lambda = xCH_4 \cdot \lambda CH_4 + xCO_2 \cdot \lambda CO_2 + x(N_2,O_2) \cdot \lambda(N_2,O_2) + xH_2 \cdot \lambda H_2 \qquad \textbf{Gl.7}$$

Die Wärmeleitfähigkeiten von $N_2$ und $O_2$ sind sehr ähnlich, so dass für das Teilgemisch $(N_2, O_2)$ näherungsweise die Wärmeleitfähigkeit von $N_2$ angesetzt wird:

$$\lambda = xCH_4 \cdot \lambda CH_4 + xCO_2 \cdot \lambda CO_2 + x(N_2,O_2) \cdot \lambda N_2 + xH_2 \cdot \lambda H_2 \qquad \textbf{Gl. 8}$$

Die Normierungsbedingung lässt sich wie folgt umstellen:

$$x(N_2, O_2) = 1 - xCH_4 - xCO_2 - xH_2 \qquad \textbf{Gl. 9}$$

Einsetzen von Gl. 9 in Gl. 8 und Auflösen liefert den Molenbruch $xH_2$

$$xH_2 = \frac{\lambda - xCH_4 \cdot (\lambda CH_4 - \lambda N_2) - xCO_2 \cdot (\lambda CO_2 - \lambda N_2) - \lambda N_2}{\lambda H_2 - \lambda N_2} \qquad \textbf{Gl. 10}$$

Die Konzentration des Wasserstoff $xH_2$ kann somit aus den Messgrößen bestimmt werden, damit ist auch die Konzentration, $x(N_2, xO_2)$ nach Gleichung 9 berechenbar.

Somit sind die Molenbrüche aller Gaskomponenten bestimmt und die Zielgrößen H, $\rho$ lassen sich analytisch berechnen.

Bei alternativer Verwendung eines nichtlinearen Ansatzes und dabei notwendiger numerischer Lösung gemäß Figur 1 werden Startwerte die Korrelation für die Stoffmengenanteile von Stickstoff $xN_2$ und Sauerstoff $xO_2$ benötigt, anhand derer ein Startwert für den Stoffmengenanteil des Wasserstoffs $H_2$ berechnet werden kann. Anhand dieser Startwerte und der gemessenen Werte kann dann iterativ durch Anpassung der Werte für Stoffmengenanteile von Stickstoff $xN_2$ und Sauerstoff $xO_2$ die Korrelationsrechnung ausgeführt und jeweils durch den Vergleich von berechneter Wärmeleitfähigkeit $\lambda$ und gemessener Wärmeleitfähigkeit $\lambda_m$ angepasst werden.

Stimmen die Werte berechneter Wärmeleitfähigkeit $\lambda$ und gemessener Wärmeleitfähigkeit $\lambda_m$ überein, liegen die tatsächlichen Stoffmengenanteile von Stickstoff $N_2$, Sauerstoff $O_2$ und Wasserstoff $H_2$ vor und daraus können dann die weiteren charakteristischen Größen des Probengases anhand der physikalischen Gesetze berechnet werden.

Zur Anpassung der Gaskenngrößen von Biogas an das Einspeisenetz können Luft und Flüssiggas (LPG=Liquid Petroleum Gas, Propan/Butan) zugemischt werden. Bei reiner Luftzumischung ist das vorstehend beschriebene Verfahren geeignet.

Bei Zumischung von LPG muss der lineare Ansatz insofern verändert werden, dass der Einfluss des Flüssiggases mit berücksichtigt werden muss, Der Beitrag von Flüssiggas kann durch eine weitere Spektralmessung erfasst werden, bei der die Wellenlänge des Infrarot-Filters auf die Absorptionsbanden der höheren Kohlenwasserstoffe (Propan und Butan) abgestimmt wird. Der Ansatz erweitert sich wie folgt:

$$xCH_4 + xCO_2 + xN_2 + xO_2 + xH_2 + xLPG = 1 \qquad \textbf{Gl. 11}$$

$$H = xCH_4 \cdot HCH_4 + xH_2 \cdot HH_2 + xLPG \cdot HLPG \qquad \textbf{Gl. 12}$$

$$\rho = xCH_4 \cdot \rho CH_4 + xCO_2 \cdot \rho CO_2 + xN_2 \cdot \rho N_2 + xO_2 \cdot \rho O_2 + xH_2 \cdot \rho H_2 + xLPG\ \rho LPG \qquad \textbf{Gl. 13}$$

Die Molenbrüche $xCH_4$, $xCO_2$ und $xLPG$ werden nach dem Beer-Lambert-Gesetz direkt aus optischen Absorptionsmessungen berechnet. Dabei müssen gegebenenfalls spezielle, vom reinen Beer-Lambert-Gesetz abweichende Kennlinien berücksichtigt werden (F1, F2, F3 sind empirische Kalibrierfunktionen):

$$xCH_4 = F1\ (ACH_0) \qquad \textbf{Gl. 14}$$

$$xCO_2 = F2\ (ACO2_0) \qquad \textbf{Gl. 15}$$

$$xLPG = F3\ (LPG_0) \qquad \textbf{Gl. 16}$$

$ACH4_0$, $ACO2_0$ und $ALPG_0$ sind die optischen Absorptionen bezogen auf einen Referenzzustand ($p_0$, $T_0$). Geeignete Absorptionsbanden liegen im infraroten Spektralbereich; typische Bereiche sind: Kohlenwasserstoffe 3, 1-3, 6 $\mu$m, insbesondere höhere Kohlenwasserstoffe (wie z.B. Propan, Butan) 3, 3- 3, 5 $\mu$m, $CO_2$ 4, 2- 4, 4 $\mu$m.

Die Konzentrationen von $H_2$, $N_2$ und $O_2$ können aus der Messung der Wärmeleitfähigkeit $\lambda$, der Normierungsbedingung Gl. 11 und folgender Modellrechnung bestimmt werden. Für die Wärmeleitfähigkeit des Gases wird ein linearer Mischungsansatz gemacht:

$$\lambda = xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2 + xLPG\ \lambda LPG \qquad \textbf{Gl. 17}$$

LPG ist typischerweise ein Gemisch aus Propan und Butan. Bei unbekannter Zusammensetzung des LPG sind die Eigenschaften dieses Gemisches, namentlich der Brennwert HLPG, die Dichte $\rho$LPG und die Wärmeleitfähigkeit $\lambda$LPG auch nicht bekannt. Es wird daher die Bestimmung aus weiteren optischen Absorptionsmessungen der Kohlenwasserstoffe angesetzt. Hierzu wird die Absorbance ggf. in weiteren Spektralbereichen gemessen und weitere Kalibrierfunktionen F4, F5 und F6 definiert:

$$HLPG = F4\left(AHLPG_0\right) \qquad \textbf{Gl. 18}$$

$$\rho LPG = F5\left(A\rho LPG_0\right) \qquad \textbf{Gl. 19}$$

$$\lambda LPG = F6\left(A\lambda LPG_0\right) \qquad \textbf{Gl. 20}$$

Da LPG im Wesentlichen nur aus Kohlenwasserstoffen besteht, sind der Brennwert und die Dichte des Gemisches stark korreliert. Alternativ zur Messung nach **Gl. 19** kann die Dichte als Funktion des Brennwertes angesetzt werden:

$$\rho LPG = F7\left(HLPG\right) \qquad \textbf{Gl. 21}$$

Die Konzentrationen xN$_2$ und xO$_2$ lassen sich nur als Summe bestimmen, die Terme werden daher zusammengefasst zu x (N$_2$, O$_2$ ) : = x (N$_2$) + x (O$_2$)

Die Wärmeleitfähigkeit des Teilgemisches (N$_2$, O$_2$) wird mit λ(N$_2$, O$_2$) bezeichnet.

Gleichung 17 lautet damit:

$$\lambda = xCH_4 \cdot \lambda CH_4 + xCO_2 \cdot \lambda CO_2 + x\,(N_2, O_2)\cdot\lambda\,(N_2, O_2) + xH_2 \cdot \lambda X_2 + xLPG \cdot \lambda LPG \qquad \text{Gl. 22}$$

Die Wärmeleitfähigkeiten von N$_2$ und O$_2$ sind sehr ähnlich, so dass für das Teilgemisch (N$_2$, O$_2$) näherungsweise die Wärmeleitfähigkeit von N$_2$ angesetzt wird:

$$\lambda = xCH_4\,\lambda CH_4 + xCO_2 \cdot \lambda CO_2 + x\,(N_2, O_2)\cdot\lambda N_2 + xH_2 \cdot \lambda H_2 + xLPG \cdot \lambda LPG \qquad \text{Gl. 23}$$

Die Normierungsbedingung lässt sich wie folgt umstellen:

$$x\,(N_2, O_2) = 1 - xCH_4 - xCO_2 - xH_2 - xLPG \qquad \text{Gl. 24}$$

Einsetzen von Gl. 24 in Gl. 23 und Auflösen liefert den Molenbruch xH$_2$

$$xH_2 = \frac{\lambda - xCH_4 \cdot (\lambda CH_4 - \lambda N_2) - xCO_2 \cdot (\lambda CO_2 - \lambda N_2) - xLPG \cdot (\lambda LPG - \lambda N_2) - \lambda N_2}{\lambda H_2 - \lambda N_2} \quad \text{Gl. 25}$$

Die Konzentration des Wasserstoff xH$_2$ kann somit aus den Messgrößen bestimmt werden, damit ist auch die Konzentration x(N$_2$, xO$_2$) nach Gl. 24 berechenbar.

Somit sind die Molenbrüche aller Gaskomponenten bestimmt und die Zielgrößen H, ρ lassen sich berechnen.

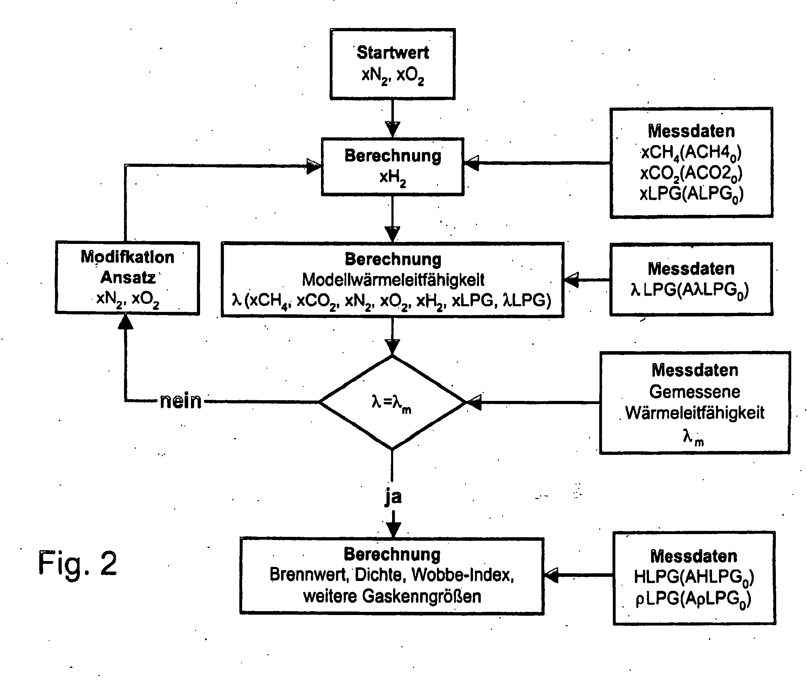

Die Figur 2 beschreibt den grundsätzlichen Ablauf des Verfahrens mit zwei Kohlen-wasserstoff-Komponenten (CH4 + Flüssiggas (LPG: C3,C4)) für die Lösung mittels numerischer Iteration bei Verwendung eines nicht-linearen Ansatzes, wie sie etwa bei der Konditionierung von Biogas durch Einspeisen von Luft und LPG vorliegen können.

Ein Ansatz zur Verfeinerung und Variation der beschriebenen Verfahren kann beispielsweise wie folgt realisiert werden:

Die Ansätze der Wärmeleitfähigkeit Gl. 6, Gl. 17 können mit Termen höherer Ordnung und mit Mischtermen verfeinert werden. Eine analytische Lösung ist dann ggf. nicht mehr möglich. Die Unbekannten xH$_2$ bzw. x(N$_2$, O$_2$) können dann durch numerische Iteration bestimmt werden.

**Literatur**

[1] Analyse und Bewertung der Nutzungsmöglichkeiten von Biomasse, Wuppertal Institut für Klima Umwelt Energie, 2005

[2] DVGW Arbeitsblatt G262: Nutzung von Gasen aus regenerativen Quellen in der öffentlichen Gasversorgung

**Patentansprüche**

1. Verfahren zur Bestimmung der Gasqualität eines zumindest teilweise mit Biogas oder aufbereitetem Biogas versetzten Probengases aufweisend die Hauptkomponenten CH$_4$, CO$_2$, N$_2$, O$_2$, H$_2$, ausgehend von einem unter Betriebsbedingungen mittels infrarotspektroskopischer Messverfahren bestimmten Spektrum des Probengases, aus dem mittels korrelativer Verfahren die Stoffmengenanteile des Probengases bestimmt und in Kenngrößen der Gesqualität umgerechnet werden,
**dadurch gekennzeichnet, dass**
die optische Absorption von Methan CH$_4$ und Kohlendioxid CO$_2$ und die Wärmeleitfähigkeit λ des Probengases und bei Zumischung von Flüssiggas LPG die optische Absorption des LPG gemessen wird,
aus der Absorption des CH$_4$ der Stoffmengenanteil xCH$_4$ und aus der Absorption des CO$_2$ der Stoffmengenanteil xCO$_2$ und bei Zumischung von Flüssiggas LPG aus der Absorption des LPG der Stoffmengenanteil xLPG bestimmt wird,

aus den Stoffmengenenteilen $xCH_4$, $xCO_2$, der Wärmeleitfähigkeit $\lambda$, und bei Zumischung von Flüssiggas LPG dem Stoffmengenanteil xLPG mittels einer Korrelationsrechnung $\lambda = F (xCH_4, xCO_2, xN_2, xO_2, xH_2)$ , bzw. $\lambda = F (xCH_4, xCO_2, xN_2, xO_2, xH_2, xLPG)$ die optisch nicht erfassten Stoffmengenanteile des Stickstoffs $xN_2$, des Sauerstoffs $xO_2$ und des Wasserstoffs $xH_2$ bestimmt werden,

woraufhin aus den so gewonnenen Stöffmengenentellen unter den Voraussetzungen, dass alle Stoffmengenanteile zusammen 100 % des Probengases ergeben und die Stoffmengenanteile von $N_2$, und $O_2$ als Summe bestimmt werden, charakteristische Parameter des Probengases berechnet werden.

2. Verfahren gemäß Anspruch 1, **dadurch gekennzeichnet, dass** als charakteristische Parameter des Probengases Brennwert, Heizwert, Dichte, Wobbe-Index, Methanzahl oder dgl. bestimmt werden.

3. Verfahren gemäß einem der vorstehenden Ansprüche, **dadurch gekennzeichnet, dass** für die Korrelation $\lambda = F (xCH_4, xCO_2, xN_2, xO_2, xH_2)$ ein linearer Ansatz aus den Stoffmengenanteilen gewählt wird:

$$\lambda = \lambda_0 + xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2$$

4. Verfahren gemäß einem der Ansprüche 1 oder 2, **dadurch gekennzeichnet, dass** bei Zumischung von LPG zu dem Probengas für die Korrelation $\lambda = F (xCH_4, xCO_2, xN_2, xO_2, xH_2, xLPG)$ ein linearer Ansatz aus den Stoffmengenanteilen gewählt wird:

$$\lambda = xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2 + xLPG \cdot \lambda LPG$$

5. Verfahren gemäß einem der Ansprüche 1 bis 4, **dadurch gekennzeichnet, dass** für die Korrelation $\lambda = F (xCH_4, xCO_2, xN_2, xO_2, xH_2)$ bzw. $\lambda = F (xCH_4, xCO_2, xN_2, xO_2, xH_2, xLPG)$ ein Polynomansatz mit Termen höherer Ordnung und Mischtermen aus den Stoffmengenanteilen gewählt wird.

6. Verfahren gemäß Anspruch 5, **dadurch gekennzeichnet, dass** die Lösung des Ansatzes der Korrelation über einen Polynomansatz durch numerische Iteration erfolgt

7. Verfahren gemäß einem der vorstehenden Ansprüche, **dadurch gekennzeichnet, dass** die gemessene Wärmeleitfähigkeit $\lambda_m$ und die berechnete Wärmeleitfähigkeit $\lambda$ durch iterative Variation und Berechnung der unbekannten Stoffmengenanteile $xN_2$, $xO_2$, $xH_2$ miteinander verglichen werden.

8. Verfahren gemäß Anspruch 7, **dadurch gekennzeichnet, dass** bei Vorliegen von Gleichheit der gemessenen Wärmeleitfähigkeit $\lambda_m$ und der berechneten Wärmeleitfähigkeit $\lambda$ die gesuchten Stoffmengenanteile bestimmt werden.

9. Verfahren gemäß einem der vorstehenden Ansprüche, **dadurch gekennzeichnet, dass** die Stoffmengenanteile $xN_2$ von $N_2$ und $xO_2$ von $O_2$ als Summe $x (N_2, O_2) := xN_2 + xO_2$ aus der Korrelation bestimmt werden.

10. Verfahren gemäß einem der vorstehenden Ansprüche, **dadurch gekennzeichnet, dass** die Wärmeleitfähigkeit des Probengases bei zwei Temperaturen ($\lambda 1$, $\lambda 2$) gemessen wird, die Wärmekapazität des Probengases bei zwei Temperaturen (C1, C2) gemessen wird und die Stoffmengenanteilen $xCH_4$, $xCO_2$, $xN_2 + xO_2$, $xH_2$ durch Lösung eines Systems von Korrelationsgleichungen $\lambda 1 = F1 (xCH_4, xCO_2, xN_2 + xO_2, xH_2)$ $\lambda 2 = F2 (xCH_4, XCO_2, xN_2 + xO_2, xH_2)$ C1 = F3 $(xCH_4, xCO_2, xN_2 + xO_2, xH_2)$ C2 = F4 $(xCH_4, xCO_2, xN_2 + xO_2, xH_2)$ bestimmt werden.

11. Verfahren gemäß einem der vorstehenden Ansprüche, **dadurch gekennzeichnet, dass** der spektrale Filter zur Bestimmung der Anteile der Kohlenwasserstoffe im Bereich von im Wesentlichen 3,33 $\mu$m arbeitet.

12. Verfahren gemäß einem der vorstehenden Ansprüche, **dadurch gekennzeichnet, dass** die Gasqualität in Bezug auf die Konzentration von Sauerstoff $O_2$ und Schwefelwasserstoff $H_2S$ überwacht wird.

13. Verfahren gemäß Anspruch 12, **dadurch gekennzeichnet, dass** die Überwachung des Probengases auf die Konzentration von Sauerstoff $O_2$ und Schwefelwasserstoff $H_2S$ mittels chemischer Sensoren, insbesondere mittels elektrochemischer Zellen durchgeführt wird.

14. Verfahren gemäß Anspruch 12, **dadurch gekennzeichnet, dass** die Überwachung des Probengases auf die Konzentration von Sauerstoff $O_2$ mittels Laserdioden durchgeführt wird.

**Claims**

1. Method for determining the gas quality of a sample gas mixed at least in part with biogas or processed biogas, having the main components $CH_4$, $CO_2$, $N_2$, $O_2$, $H_2$, proceeding from a spectrum of the sample gas determined by means of infrared- spectroscopy measurement methods, under operating conditions, from which the mole ratios of the sample gas are determined by means of correlative methods, and converted to characteristic variables of the gas quality, **characterized in that**
   the optical absorption of methane $CH_4$ and carbon dioxide $CO_2$ and the heat conductivity $\lambda$ of the sample gas, and, if liquid gas LPG is mixed in, the optical absorption of the LPG are measured,
   the mole ratio $xCH_4$ is determined from the absorption of the $CH_4$, the mole ratio $xCO_2$ is determined from the absorption of the $CO_2$, and, if liquid gas LPG is mixed in, the mole ratio $xLPG$ is determined from the absorption of the LPG,
   the mole ratios of nitrogen $xN_2$, of oxygen $xO_2$ and of hydrogen $xH_2$ that are not detected optically are determined from the mole ratios $xCH_4$, $xCO_2$ and the heat conductivity $\lambda$, and, if liquid gas LPG is mixed in, from the mole ratio $xLPG$, by means of a correlation calculation $\lambda = F\ (xCH_4, xCO_2, xN_2, xO_2, xH_2)$ , or $\lambda = F\ (xCH_4, xCO_2, xN_2, xO_2, xH_2, xLPG)$ ,
   whereupon characteristic parameters of the sample gas are calculated from the mole ratios obtained in this way, under the prerequisites that all the mole ratios together amount to 100% of the sample gas, and that the mole ratios of $N_2$ und $O_2$ are determined as a sum.

2. Method according to claim 1, **characterized in that** fuel value, heating value, density, Wobbe index, methane number, or the like are determined as characteristic parameters of the sample gas.

3. Method according to one of the preceding claims, **characterized in that** a linear formula for the correlation $\lambda = F\ (xCH_4, xCO_2, xN_2, xO_2, xH_2)$ is selected from among the mole ratios:

$$\lambda = \lambda_0 + xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2$$

4. Method according to one of claims 1 or 2, **characterized in that** when LPG is mixed into the sample gas, a linear formula for the correlation $\lambda = F\ (xCH_4, xCO_2, xN_2, xO_2, xH_2, xLPG)$ is selected from among the mole ratios:

$$\lambda = xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2 + xLPG \cdot \lambda LPG$$

5. Method according to one of claims 1 to 4, **characterized in that** a polynomial formula for the correlation $\lambda = F\ (xCH_4, xCO_2, xN_2, xO_2, xH_2)$ or $\lambda = F\ (xCH_4, xCO_2, xN_2, xO_2, xH_2, xLPG)$ is selected from among the mole ratios, with terms of a higher order and mixed terms.

6. Method according to claim 5, **characterized in that** the solution of the formula of the correlation takes place by way of a polynomial formula, by means of numerical iteration.

7. Method according to one of the preceding claims, **characterized in that** the measured heat conductivity $\lambda_m$ and the calculated heat conductivity $\lambda$ are compared with one another by means of iterative variation and calculation of the unknown mole ratios $xN_2$, $xO_2$, $xH_2$.

8. Method according to claim 7, **characterized in that** the mole ratios being sought are determined when the measured heat conductivity $\lambda_m$ and the calculated heat conductivity $\lambda$ are equal.

9. Method according to one of the preceding claims, **characterized in that** the mole ratios $xN_2$ of $N_2$ and $xO_2$ of $O_2$ are determined from the correlation as a sum $x\ (N_2, O_2)\ : = xN_2 + xO_2$.

10. Method according to one of the preceding claims, **characterized in that** the heat conductivity of the sample gas is measured at two temperatures ($\lambda 1$, $\lambda 2$) , the heat capacity of the sample gas is measured at two temperatures (C1, C2) , and the mole ratios $xCH_4$, $xCO_2$, $xN_2+xO_2$, $xH_2$ are determined by means of solving a system of correlation equations

$$\lambda 1 = F1\ (xCH_4, xCO_2, xN_2 + xO_2, xH_2)$$

$$\lambda 2 = F2\ (xCH_4, xCO_2, xN_2 + xO_2, xH_2)\quad C1 = F3\ (xCH_4, xCO_2, xN_2 + xO_2, xH_2)\quad C2 = F4\ (xCH_4, xCO_2, xN_2 + xO_2, xH_2)\ .$$

**11.** Method according to one of the preceding claims, **characterized in that** the spectral filter for determining the proportions of the hydrocarbons works in the range of essentially 3.33 μm.

**12.** Method according to one of the preceding claims, **characterized in that** the gas quality is monitored with regard to the concentration of oxygen $O_2$ and hydrogen sulfide $H_2S$.

**13.** Method according to claim 12, **characterized in that** monitoring of the sample gas with regard to the concentration of oxygen $O_2$ and hydrogen sulfide $H_2S$ is carried out by means of chemical sensors, particularly by means of electrochemical cells.

**14.** Method according to claim 12, **characterized in that** monitoring of the sample gas with regard to the concentration of oxygen $O_2$ is carried out by means of laser diodes.

**Revendications**

**1.** Procédé servant à déterminer la qualité du gaz d'un échantillon de gaz, mélangé au moins en partie avec un biogaz ou un biogaz valorisé et présentant les composants principaux $CH_4$, $CO_2$, $N_2$, $O_2$, $H_2$, à partir d'un spectre de l'échantillon de gaz déterminé dans des conditions de fonctionnement au moyen d'un procédé de mesure par spectroscopie infrarouge, à partir duquel spectre on détermine, au moyen d'un procédé de corrélation, les fractions molaires de l'échantillon de gaz que l'on convertit en caractéristiques de la qualité du gaz,
**caractérisé en ce**
qu'on mesure l'absorption optique du méthane $CH_4$ et du dioxyde de carbone $CO_2$ et la conductivité thermique $\lambda$ de l'échantillon de gaz et, lors du mélange de gaz de pétrole liquéfié GPL, l'absorption optique de ce dernier,
qu'on détermine à partir de l'absorption du $CH_4$ la fraction molaire $xCH_4$, et à partir de l'absorption du $CO_2$ la fraction molaire $xCO_2$, et, lors du mélange de gaz de pétrole liquéfié GPL, à partir de l'absorption du GPL la fraction molaire xGPL,
qu'on détermine, à partir des fractions molaires $xCH_4$, $XCO_2$, à partir de la conductivité thermique $\lambda$, et, lors du mélange de gaz de pétrole liquéfié GPL, à partir de la fraction molaire xGPL, au moyen d'un calcul de corrélation $\lambda = F(xCH_4, xCO_2, xN_2, xO_2, xH_2)$ ou $\lambda = F(xCH_4, xCO_2, xN_2, xO_2, xH_2, xLPG)$, les fractions molaires non détectées optiquement de l'azote $xN_2$, de l'oxygène $xO_2$ et de l'hydrogène $xH_2$,
qu'on calcule ensuite, à partir des fractions molaires ainsi obtenues à condition que toutes les fractions molaires représentent ensemble 100 % du gaz d'échantillon et que les fractions molaires de $N_2$ et $O_2$ soient déterminées comme une somme, des paramètres caractéristiques du gaz d'échantillon.

**2.** Procédé selon la revendication 1, **caractérisé en ce qu'**on détermine comme paramètres caractéristiques du gaz d'échantillon, le pouvoir calorifique, la densité, l'indice Wobbe, l'indice méthane ou similaire.

**3.** Procédé selon l'une quelconque des revendications précédentes, **caractérisé en ce que** pour la corrélation $\lambda = F(xCH_4, xCO_2 xN_2, XO_2, xH_2)$, on choisit une approche linéaire à partir des fractions molaires : $\lambda = \lambda_0 + xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2$

**4.** Procédé selon l'une quelconque des revendications 1 ou 2, **caractérisé en ce que** lors du mélange de GPL au gaz d'échantillon pour la corrélation $\lambda = F(xCH_4, xCO_2, xN_2, xO_2, xH_2, xLPG)$, on choisit une approche linéaire à partir des fractions molaires :

$$\lambda = xCH_4 \cdot \lambda CH_4 + xCO_2 \cdot \lambda CO_2 + xN_2 \cdot \lambda N_2 + xO_2 \cdot \lambda O_2 + xH_2 \cdot \lambda H_2 + xLPG \cdot \lambda LPG$$

**5.** Procédé selon l'une quelconque des revendications 1 à 4, **caractérisé en ce que** pour la corrélation $\lambda = F(xCH_4, xCO_2, xN_2, xO_2, xH_2)$ ou $\lambda = F(xCH_4, xCO_2, xN_2, xO_2, xH_2, xLPG)$, on choisit une approche polynomiale avec des termes de niveau supérieur et des termes de mélange à partir des fractions molaires.

**6.** Procédé selon la revendication 5, **caractérisé en ce que** l'approche de la corrélation par une approche polynomiale est résolue par une itération numérique.

**7.** Procédé selon l'une quelconque des revendications précédentes, **caractérisé en ce que** la conductivité thermique $\lambda_m$ mesurée et la conductivité thermique $\lambda$ calculée sont comparées l'une à l'autre par une variation itérative et par un calcul des fractions molaires inconnues $xN_2$, $xO_2$, $xH_2$.

8. Procédé selon la revendication 7, **caractérisé en ce que** lorsque la conductivité thermique $\lambda_m$ mesurée et la conductivité thermique $\lambda$ calculée sont égales, on détermine les fractions molaires recherchées.

9. Procédé selon l'une quelconque des revendications précédentes, **caractérisé en ce que** les fractions molaires $xN_2$ de $N_2$ et $xO_2$ de $O_2$ sont déterminées comme somme $x(N_2, O_2) = xN_2 + xO_2$ à partir de la corrélation.

10. Procédé selon l'une quelconque des revendications précédentes, **caractérisé en ce que** la conductivité thermique du gaz d'échantillon est mesurée à deux températures ($\lambda 1$, $\lambda 2$), **en ce que** la capacité calorifique du gaz d'échantillon est mesurée à deux températures (C1, C2), et **en ce qu'**on détermine les fractions molaires $xCH_4$, $xCO_2$, $xN_2 + xO_2$, $xH_2$ par la résolution d'un système d'équations de corrélation

$$\lambda 1 = F1(xCH_4, xCO_2, xN_2 + xO_2, xH_2)$$

$$\lambda 2 = F2(xCH_4, xCO_2, xN_2 + xO_2, xH_2)$$

$$C1 = F3(xCH_4, xCO_2, xN_2 + xO_2, xH_2)$$

$$C2 = F4(xCH_4, xCO_2, xN_2 + xO_2, xH_2)$$

11. Procédé selon l'une quelconque des revendications précédentes, **caractérisé en ce que** le filtre spectral servant à déterminer les fractions des hydrocarbures fonctionne de manière centrée sur essentiellement 3,33 $\mu$m.

12. Procédé selon l'une quelconque des revendications précédentes, **caractérisé en ce que** la qualité du gaz en termes de concentration d'oxygène $O_2$ et de sulfure d'hydrogène $H_2S$ est surveillée.

13. Procédé selon la revendication 12, **caractérisé en ce que** la surveillance de la concentration d'oxygène $O_2$ et de sulfure d'hydrogène $H_2S$ de l'échantillon de gaz est effectuée au moyen de capteurs chimiques, en particulier au moyen de cellules électrochimiques.

14. Procédé selon la revendication 12, **caractérisé en ce que** la surveillance de la concentration d'oxygène $O_2$ du gaz d'échantillon est effectuée au moyen de diodes laser.

**Startwert**
$xN_2$, $xO_2$

**Messdaten**
$xCH_4(ACH4_0)$
$xCO_2(ACO2_0)$

**Berechnung**
$xH_2$

**Modifikation Ansatz**
$xN_2$, $xO_2$

**Berechnung**
Modellwärmeleitfähigkeit
$\lambda(xCH_4, xCO_2, xN_2, xO_2, xH_2)$

$\lambda = \lambda_m$

**Messdaten**
Gemessene
Wärmeleitfähigkeit
$\lambda_m$

nein

ja

**Berechnung**
Brennwert, Dichte, Wobbe-Index,
weitere Gaskenngrößen

Fig. 1

**Fig. 2**

Startwert
$xN_2$, $xO_2$

Messdaten
$xCH_4(ACH4_0)$
$xCO_2(ACO2_0)$
$xLPG(ALPG_0)$

Berechnung
$xH_2$

Modifkation
Ansatz
$xN_2$, $xO_2$

Berechnung
Modellwärmeleitfähigkeit
$\lambda(xCH_4, xCO_2, xN_2, xO_2, xH_2, xLPG, \lambda LPG)$

Messdaten
$\lambda LPG(A\lambda LPG_0)$

Messdaten
Gemessene
Wärmeleitfähigkeit
$\lambda_m$

nein

$\lambda = \lambda_m$

ja

Berechnung
Brennwert, Dichte, Wobbe-Index,
weitere Gaskenngrößen

Messdaten
$HLPG(AHLPG_0)$
$\rho LPG(A\rho LPG_0)$

14

**IN DER BESCHREIBUNG AUFGEFÜHRTE DOKUMENTE**

**In der Beschreibung aufgeführte Patentdokumente**

- EP 1141677 B1
- EP 1147396 B1
- WO 03062618 A1
- US 2004195531 A1

**In der Beschreibung aufgeführte Nicht-Patentliteratur**

- Analyse und Bewertung der Nutzungsmöglichkeiten von Biomasse. *Wuppertal Institut für Klima Umwelt Energie,* 2005